(19) Europäisches Patentamt
European Patent Office
Office européen des brevets

(11) **EP 4 629 248 A1**

(12) **EUROPEAN PATENT APPLICATION**
published in accordance with Art. 153(4) EPC

(43) Date of publication:
08.10.2025 Bulletin 2025/41

(21) Application number: 24837525.5

(22) Date of filing: 20.02.2024

(51) International Patent Classification (IPC):
*G16B 30/00* (2019.01)     *G16B 5/20* (2019.01)
*G16B 40/00* (2019.01)     *G06N 3/08* (2023.01)
*G16B 15/20* (2019.01)     *G16B 40/20* (2019.01)

(52) Cooperative Patent Classification (CPC):
**G16B 15/20; G16B 30/00; G16B 40/20**

(86) International application number:
**PCT/CN2024/077839**

(87) International publication number:
**WO 2025/175491 (28.08.2025 Gazette 2025/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Beijing Youzhuju Network Technology Co., Ltd.
Beijing 101299 (CN)**
• **Lemon Inc.
Grand Cayman, KY1-1205 (KY)**

(72) Inventors:
• **ZHENG, Zaixiang
Beijing 100028 (CN)**
• **WANG, Xinyou
Beijing 100028 (CN)**
• **YE, Fei
Beijing 100028 (CN)**
• **XUE, Dongyu
Beijing 100028 (CN)**
• **GU, Quanquan
Los Angeles, California 90066 (US)**

(74) Representative: **Grünecker Patent- und
Rechtsanwälte
PartG mbB
Leopoldstraße 4
80802 München (DE)**

(54) **PROTEIN SEQUENCE GENERATION AND REPRESENTATION LEARNING**

(57)   Embodiments of the present disclosure relate to protein sequence generation and representation learning. The method includes: obtaining a target model which is based on a discrete diffusion probability model and a language model structure and is pre-trained using a real protein sequence set; and performing, using the target model, at least one of the following: generating a first target protein sequence using the target model based at least on an input sequence in a form of protein sequence, or extracting a sequence feature representation corresponding to a second target protein sequence using the target model based on the second target protein sequence.

FIG. 2

**Description**

**FIELD**

Example embodiments of the present disclosure generally relate to the field of computers, and in particular to protein sequence generation and representation learning.

**BACKGROUND**

Proteins are three-dimensional (3D) folded linear sequences of amino acid residues. Proteins perform many important vital activities in organisms and play a pivotal role in regulating various biological functions, including transcription, translation, signaling and the control of cell cycle, and the like. With developments of data-driven deep learning technology, in the field of protein analysis, it has gradually evolved from traditional methods to using models to learn how to understand and design proteins. It is desirable to develop powerful models to accomplish various tasks of interest in the field of protein research.

**SUMMARY**

In a first aspect of the present disclosure, there is provided a method for information processing. The method comprises the following steps: obtaining a target model which is based on a discrete diffusion probability model and a language model structure and is pre-trained using a real protein sequence set; and performing, using the target model, at least one of the following: generating a first target protein sequence using the target model based at least on an input sequence in a form of protein sequence, or extracting a sequence feature representation corresponding to a second target protein sequence using the target model based on the second target protein sequence.

In a second aspect of the present disclosure, an apparatus for information processing is provided. The device comprises a model obtaining module configured to obtain a target model which is based on a discrete diffusion probability model and a language model structure and is pre-trained using a real protein sequence set; and a model performing module configured to perform, using the target model, at least one of the following: generating a first target protein sequence using the target model based at least on an input sequence in a form of protein sequence, or extracting a sequence feature representation corresponding to a second target protein sequence using the target model based on the second target protein sequence.

In a third aspect of the present disclosure, an electronic device is provided. The electronic device includes at least one processing unit; and at least one memory coupled to the at least one processing unit and storing instructions for execution by the at least one processing unit, the instructions, when executed by the at least one processing unit, causing the electronic device to perform the method of the first aspect of the present disclosure.

In a fourth aspect of the present disclosure, a computer-readable storage medium is provided. The computer-readable storage medium has stored thereon a computer program executable by a processor to perform the method according to the first aspect of the present disclosure.

In a fifth aspect of the present disclosure, a computer program product is provided. The computer program product is tangibly stored in a computer storage medium and includes computer-executable instructions that, when executed by a device, cause the device to perform the method according to the first aspect of the present disclosure.

It should be understood that the content described in the summary section is not intended to limit the key features or important features of the embodiments of the present disclosure, nor is it intended to limit the scope of the present disclosure. Other features of the present disclosure will become readily understood from the following description.

**BRIEF DESCRIPTION OF DRAWINGS**

The above and other features, advantages, and aspects of the various implementations of the present disclosure will become more apparent from the following detailed description taken in conjunction with the accompanying drawings. In the drawings, the same or similar reference numbers refer to the same or similar elements, wherein:

FIG. 1 illustrates a schematic diagram of an example environment in which embodiments of the present disclosure can be implemented;
FIG. 2 shows a schematic diagram of a training architecture for a protein sequence generation and representation learning model according to some embodiments of the present disclosure;
FIG. 3 illustrates an example of extracting a sequence feature representation of a protein sequence with a model according to some embodiments of the present disclosure;
FIG. 4A illustrates an example of a conditioning generation task conditioned on partial sequences according to some embodiments of the present disclosure;

FIG. 4B illustrates an example of a conditioning generation task conditioned on a secondary structure according to some embodiments of the present disclosure;

FIG. 4C illustrates an example of a controllable sequence generation task with guidance information according to some embodiments of the present disclosure;

FIG. 5 shows a flowchart of a processing process for protein related information according to some embodiments of the present disclosure;

FIG. 6 shows a block diagram of a processing apparatus for protein related information according to some embodiments of the present disclosure; and

FIG. 7 illustrates a block diagram of an electronic device in which one or more embodiments of the present disclosure may be implemented.

## DETAILED DESCRIPTION

Embodiments of the present disclosure will be described in more detail below with reference to the accompanying drawings. While certain embodiments of the present disclosure are shown in the accompanying drawings, it should be understood that the present disclosure may be implemented in various forms, and should not be construed as limited to the embodiments set forth herein, but rather, these embodiments are provided for a more thorough and complete understanding of the present disclosure. It should be understood that the drawings and embodiments of the present disclosure are for exemplary purposes only and are not intended to limit the scope of the present disclosure.

In the description of embodiments of the present disclosure, the terms "comprise" and the like should be understood to include "comprising but not limited to". The term "based on" should be understood as "based at least in part on". The terms "one embodiment" or "the embodiment" should be understood as "at least one embodiment". The term "some embodiments" should be understood as "at least some embodiments". Other explicit and implicit definitions may also be included below.

As used herein, unless explicitly stated, "in response to A" performs one step and does not imply that this step is performed immediately after "A", but may include one or more intermediate steps.

It may be understood that data involved in the technical solution (including but not limited to the data itself, the obtaining, using, storing or deleting of the data) should follow requirements of the corresponding laws and regulations and related regulations.

It can be understood that before using the technical solutions in embodiments of the present disclosure, a relevant user should be informed of the types, use ranges, usage scenarios, and the like of the information related to the present disclosure in an appropriate manner according to relevant laws and regulations, and the authorization of the related user may be obtained, where the relevant users may include any type of rights body, such as individuals, businesses, and groups.

For example, in response to that an active request of a user is received, prompt information is sent to the related user to explicitly prompt the related that operations requested to be performed will need to obtain and use information of the related user, so that the related user can autonomously select whether to provide information to software or hardware executing the operations of the technical solution of the present disclosure according to the prompt information.

As an optional but non-limiting implementation, in response to receiving an active request of a related user, a manner of sending prompt information to the related user may be, for example, a pop-up window, and prompt information may be presented in a text manner in the pop-up window. In addition, the pop-up window may further carry a selection control for the user to select "agree" or "not agree" to provide information to the electronic device.

It may be understood that the foregoing notification and obtaining a user authorization process are merely illustrative, and do not constitute a limitation on implementations of the present disclosure, and other manners of meeting related laws and regulations may also be applied to implementations of the present disclosure. According to the present discourse, enabling the digital assistant related function, the acquired data, the data processing and storage mode and the like are authorized in advance by the user and other right main bodies associated with the user, and comply with the agreement of the related laws and regulations and the right main body protocol rules.

As used herein, the term "model" may learn an association relationship between respective inputs and outputs from training data, such that a corresponding output may be generated for a given input after training is complete. The generation of the model may be based on machine learning techniques. Deep learning is a machine learning algorithm that processes inputs and provides corresponding outputs using a multi-layer processing unit. The neural network model is one example of a deep learning-based model. As used herein, the "model" may also be referred to as a "machine learning model," a "learning model," a "machine learning network," or a "learning network," which terms are used interchangeably herein.

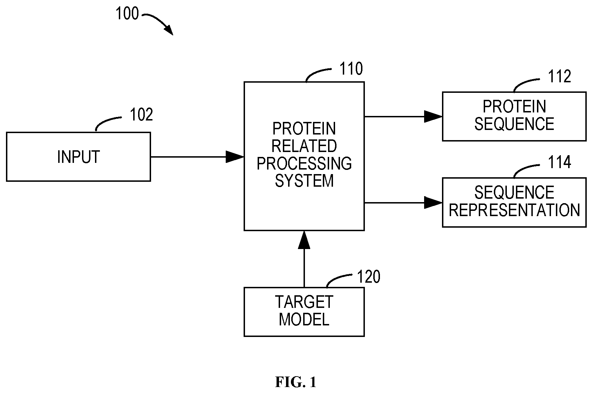

FIG. 1 illustrates a schematic diagram of an example environment 100 in which embodiments of the present disclosure can be implemented. In environment 100, a protein-related processing system 110 may utilize a target model 120 to perform protein-related analysis tasks based on a specified input 102. In protein-related analysis, protein sequence prediction, protein structure prediction, representation learning of protein sequences and the like are all tasks with practical meanings. In some implementations, the processing system 110 may generate a protein sequence using the target model 120 based on the input 102. In the processing system 110, the sequence representation corresponding to the protein sequence may be represented using the target model 120 based on the input 102 (for example, the input protein sequence). On the basis of accurate sequence representation, various understanding tasks may be performed, including a classification of protein sequences, a classification of residues in proteins, a protein sequence regression, a protein contact point prediction, and the like.

In FIG. 1, the processing system 110 may be any type of device having computing capability, including a terminal device or a server device. The terminal device may be any type of mobile terminal, fixed terminal, or portable terminal, including a mobile handset, a desktop computer, a laptop computer, a notebook computer, a netbook computer, a tablet computer, a media computer, a multimedia tablet, a personal communication system (PCS) device, a personal navigation device, a personal digital assistant (PDA), an audio/video player, a digital camera/camcorder, a positioning device, a television receiver, a radio broadcast receiver, an e-book device, a gaming device, or any combination of the foregoing, including accessories and peripherals of these devices, or any combination thereof. The server device may include, for example, a computing system/server, such as a mainframe, an edge computing node, a computing device in a cloud environment, or the like.

It should be understood that the structure and function of the environment 100 is described for example purposes only and does not imply any limitation to the scope of the present disclosure.

In the field of deep learning, language model (LM) achieves remarkable progress in the field of natural language understanding. Given the similarity between a protein sequence and a human language, it has been discussed in the field of protein research that various tasks are implemented using a language model, including protein-related prediction tasks (for example, detecting functional properties, predicting a protein structure from a single sequence without the need to evolve homologous chromosomes), protein-related generation tasks (for example, redesigning an optimized protein sequence for given protein backbone structures, or synthesizing completely new protein sequences).

The language model is intended to estimate the underlying distribution $x \sim q(x)$ of sequence data of interest (for example, text or protein sequence) by learning a probabilistic model $p_\theta(x)$. The language model may be parameterized by a neural network (for example, Transform-based neural network) as $\theta$. In language modeling for a protein sequence, sequence data to be processed may be represented as $x = (x_1, x_2, ..., x_L) \in \{0,1\}^{L \times |V|}$ that is composed of L elements. $\mathcal{V}$ is a vocabulary of discrete data. For a protein sequence, V includes a plurality of amino acids for constituting a protein, for example, may be composed of 20 different amino acids, expressed as $\mathcal{V}$ = {1, ..., 20}.

The current language model is implemented by using different model structures in prediction tasks and generation tasks. For a prediction task (which requires understanding capability), a widely used pre-training goal is a mask prediction. For the mask prediction, a masked language model (MLM) can detect the bidirectional receptive field of the sequence to obtain the understanding capability. The bidirectional receptive field is able to take into account both left-to-right and right-to-left directions, thus taking into account the context of the left and right sides, predicting the masked sequence (i.e., amino acid) by mask predicted self-coding scheme. The pre-training goal for mask prediction may be represented as follows:

$$\mathbb{E}_{q(\mathbf{x})} \log p_\theta(\mathbf{x}) = \mathbb{E}_{q(\mathbf{x})} \sum_{1 \leq i \leq L} b_i \cdot \log p_\theta(x_i | \bar{\mathbf{x}}_{\mathrm{m}}) \tag{1}$$

where $b_i = 1_{\bar{x}_i = [X]}$ is a mask sequence (for example, a scaling mask and the like) of a mask that is performed on a sequence x with a special mask symbol. The mask sequence after masking is represented as $\bar{\mathbf{x}}_{\mathrm{m}}$. This pre-training goal is based on a per-token conditional independence assumption. Mask prediction can obtain a better model performance for a sequence understanding task of natural language and protein sequences.

For generation tasks, widely used pre-training goals are autoregressive training. Autoregressive training uses probabilistic chain rules to perform a serialization factor decomposition on sequences. Model learning is then performed by maximizing the log-likelihood values of the model on a training dataset, which is represented as follows:

$$\mathbb{E}_{q(\mathbf{x})} \log p_\theta(\mathbf{x}) = \mathbb{E}_{q(\mathbf{x})} \sum_{1 < i < L} \log p_\theta(x_i | \mathbf{x}_{<i}) \tag{2}$$

where a causal mask is used to ensure sequence dependent structures. In order to sample the autoregressive model, L iterative steps need to be performed in a strict left-to-right unidirectional manner from $x_1 \sim p_\theta(x_1)$, $x_2 \sim p_\theta(x_2 | x_1)$ to $x_L \sim p(x_L | x_1, ..., x_{L-1})$.

By analyzing language models currently employed in the field of natural language processing, the inventors have the following observations.

In mask prediction, it is difficult to perform sequence generation tasks because the model lacks the configuration of generative modeling. Moreover, bidirectionality nature in mask prediction makes it difficult to be applied to sequence generation. In addition, proteins are structured macromolecules rather than simple linear sequences. Therefore, the autoregressive language model only considers a sequence context of a single direction in natural language processing. In protein-related tasks, the autoregressive language model is limited by the inability to capture complex global interactions of amino acids, and thus satisfactory results in terms of protein generation and prediction capabilities cannot be achieved.

The autoregressive language model can have a sequence generation capability, but cannot well understand the sequence data. In the generation task, the model needs to continuously approach the underlying data distribution, thereby creating a new output sample. Therefore, it is desirable to generate a model to ensure a deep understanding of the data at the same time.

In embodiments of the present disclosure, it is desirable to be able to propose a unified universal model, with both prediction and generation capabilities. In the generation task, the universal model is a powerful extensible generative model and can well capture protein sequences. In the prediction task, the universal model has a bidirectional receptive field and can well model the global interaction at the residue level.

In embodiments of the present disclosure, a language model based on a discrete diffusion probability model structure is proposed, referred to as a diffusion protein language model (DPLM). The model is pre-trained using a large amount of real protein sequence data to enable learning of knowledge in the evolutionary level protein sequence in the real world. The model simultaneously has the generation capability of the language model and the understanding capability of the diffusion model. The discrete diffusion probability model structure may have a generative generalization capability for language modeling. During pre-training, the model is subjected to denoising at different noise levels from the input protein sequence as training data according to the training principle of the diffusion model, thereby obtaining a plurality of capabilities for understanding and outputting the protein. After pre-training, the model can be used for protein sequence generation and provide accurate sequence feature representation of the protein sequence for various prediction tasks downstream.

Some example embodiments of the present disclosure will be described below with continued reference to the accompanying drawings.

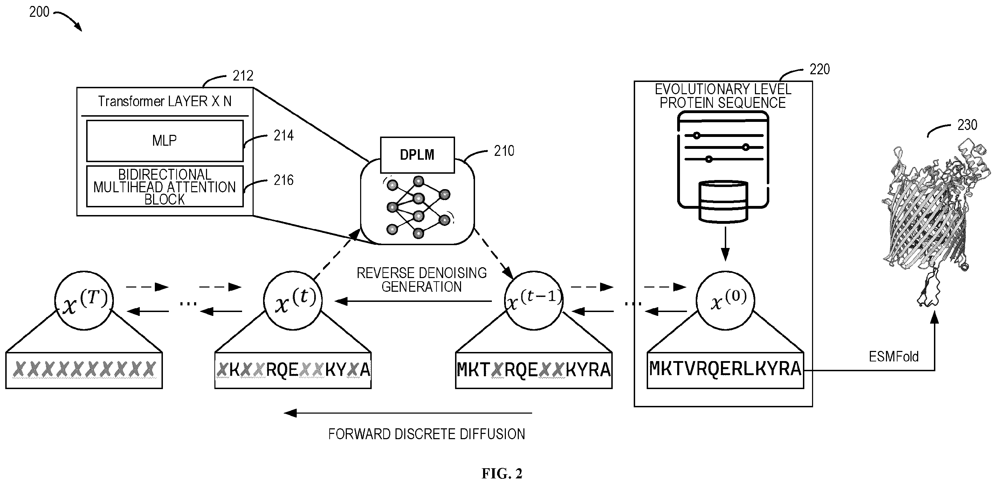

FIG. 2 shows a schematic diagram of a training architecture 200 for a protein sequence generation and representation learning model according to some embodiments of the present disclosure.

In the architecture 200, a diffusion protein language model (DPLM) 210 (also sometimes referred to herein as a target model) is constructed based on discrete diffusion probability model structures and language model structures. Specifically, the DPLM 210 may include multiple Transformer layers 212 used in a language model. Each Transformer layer 212 may include a multi-layer perceptron (MLP) 214 and a multi-head attention block 216. The Transformer layer also has other structural designs besides the structure shown in FIG. 2. On a basis of structure of the language model, the DPLM 210 also performs a data processing process by diffusion probability models, i.e., a discrete diffusion probability model.

As previously mentioned, in language modeling for a protein sequence, the sequence data to be processed may be represented as $x = (x_1, x_2, ..., x_L) \in \{0, 1\}^{L \times |V|}$, which is composed of $L$ elements. V is a vocabulary of discrete data. For a protein sequence, $\mathcal{V}$ includes a plurality of amino acids for constituting a protein, for example, may be composed of 20 different amino acids, expressed as $\mathcal{V} = \{1, ..., 20\}$. Thus, for the DPLM 210, an element (also referred to as token) corresponding to each position in the input sequence and the output sequence may indicate any one amino acid in the vocabulary of the amino acid, or may indicate a special symbol (for example, [X], the symbol does not represent any amino acid).

For a better understanding, a diffusion probability model will be briefly introduced below, and then a discrete diffusion probability model is introduced.

The diffusion probability model is a type of generative model, but its data generation process is based on a pair of Markov processes, i.e., a forward diffusion process and a backward denoising process. The forward diffusion process (represented as: $q(\mathbf{x}^{(1:T)}|\mathbf{x}^{(0)}) = \prod_{t=1}^{T} q(\mathbf{x}^{(t)}|\mathbf{x}^{(t-1)})$ ) gradually perturbs the data $x^{(0)} \sim q(x^{(0)})$ into a stationary noise distribution $x^{(T)} \sim q_{noise}$ with $T$ increasingly noisy steps $x^{(1:T)} = x_1,...,x^{(t-1)},x^{(t)},...,x^{(T)}$. Through model training, the learn backward denoising process (represented as: $p_\theta(\mathbf{x}^{(0:T)}) = p(\mathbf{x}^{(t)}) \prod_{t=1}^{T} p_\theta(\mathbf{x}^{(t-1)}|\mathbf{x}^{(t)})$ ) performs an inverse process, to gradually denoise the samples towards the data distribution and obtain data $x^{(0)} \sim q(x^{(0)})$. Thus, the backward denoising process may correspond to a desired data modeling process, and finally obtain desired data.

In some implementations, to fit the model (denoted as: $p_\theta(x^{(0)})$) to the data distribution $q(x^{(0)})$, the learning of the backward denoising process is typically implemented by optimizing the variational constraint of the log-likelihood, which may be represented as follows:

$$\mathbb{E}_{q(\mathbf{x}^{(0)})}\left[\log p_\theta(\mathbf{x}^{(0)})\right] \geq \mathbb{E}_{q(\mathbf{x}^{(0:T)})}\left[\log \frac{p_\theta(\mathbf{x}^{(0:T)})}{q(\mathbf{x}^{(1:T)}|\mathbf{x}^{(0)})}\right] \qquad (3)$$

$$= \mathbb{E}_{q(\mathbf{x}^{(0)})}\left[\log p_\theta(\mathbf{x}^{(0)}|\mathbf{x}^{(1)}) + \text{const.}\right.$$

$$\left.\underbrace{\sum_{t=2}^T -\text{KL}\left[q(\mathbf{x}^{(t-1)}|\mathbf{x}^{(t)}, \mathbf{x}^{(0)})\|p_\theta(\mathbf{x}^{(t-1)}|\mathbf{x}^{(t)})\right]}_{\mathcal{J}_t}\right]$$

after the learning is completed, the model of the backward denoising process can first sample from the noise distribution $q_{\text{noise}}$ ($x^{(T)}$), and followed by iterative denoising with $p_\theta(x^{(t-1)}|x^{(t)})$ until the desired data is obtained.

In the diffusion probability model, continuous diffusion with Gaussian perturbation kernel has demonstrated impressive performance in generating continuous data in Euclidean space, and the more general Riemannian manifolds. Given that the bidirectional receptive field of the diffusion model is ideally suited for modeling residue-wise global interactions, it is believed that the diffusion model is suitable for understanding of the protein sequence. On the other hand, the language model can realize diffusible effective sequence learning, and is suitable for sequence generation of proteins. Thus, in embodiments of the present disclosure, it is desirable to obtain a model that simultaneously has an understanding (prediction) capability and sequence generation capability of a protein sequence.

A direct use of continuous diffusion, however, is not necessarily the best choice for modeling discrete sequence data. This is because the protein sequence belongs to discrete data, and the Gaussian diffusion hardly models the discrete nature of sequence data in embedding representation space, resulting in *pitfall of discreteness.* To this end, in embodiments of the present disclosure, for the DPLM 210 for protein sequence generation and representation learning, a discrete diffusion probability model structure operating directly in a discrete state space is proposed to model a protein sequence. In addition, the discrete diffusion probability model is also performed on the structure of the language model.

Specifically, it is assumed that Cat(**x**; **p**) is a categorical distribution on protein sequence **x** parameterized by a vector on **p** on ($|\mathcal{V}|$-1) dimensional probability simplex (this belongs to a probability distribution for describing a probability distribution of the random variables having a finite number of categories). The forward diffusion process of the discrete diffusion probability model defines a Markov process governed by the transition kernel, represented as follows:

$$q(\mathbf{x}^{(t)}|\mathbf{x}^{(t-1)}) = \text{Cat}\left(\mathbf{x}^{(t)}; \beta_t \mathbf{x}^{(t-1)} + (1-\beta_t)\mathbf{q}_{\text{noise}}\right) \qquad (4)$$

where $\mathbf{q}_{\text{noise}}$ is the probability vector of stationary distribution $q_{\text{noise}}(x^{(t)})$, i.e., $q(x^{(t)}) = \text{Cat}(x^{(t)}; p = q_{\text{noise}})$ and $0 \ll \beta_t < 1$ is the noise schedule controlling the degree of corruption at timestep $t$. Thus, the distribution of corrupted sequence data $x^{(t)}$ given its original protein sequence $x^{(0)}$ has a closed-form expression:

$$q(\mathbf{x}^{(t)}|\mathbf{x}^{(0)}) = \text{Cat}\left(\mathbf{x}^{(t)}; \alpha_t \mathbf{x}^{(0)} + (1-\alpha_t)\mathbf{q}_{\text{noise}}\right) \qquad (5)$$

where $\alpha_t = \prod_{i=1}^t \beta_i$ such that $\lim_{t \to T} \alpha_t \to 0$, which preserves no information of original protein sequence from the data and converges to the stationary distribution $\mathbf{q}_{\text{noise}}$ at timestep $T$. This represents that an entire diffusion process is a convex combination of data (original protein sequence) and stationary noise prior distribution.

Different stationary noise distributions $\mathbf{q}_{\text{noise}}$ may lead to different formulations of discrete diffusion models. In some embodiments, discrete diffusion with limitations is employed: if $\mathbf{x}^{(t)} = [X]$, $q(\mathbf{x}^{(t)}) = 1$; if $\mathbf{x}^{(t)} \neq [X]$, $q(\mathbf{x}^{(t)}) = 0$, where $[X]$ represents an absorbing state, no specific amino acid is indicated in the protein sequence representation. The above formula (5) results in sequence data $\mathbf{x}^{(t)}$ at the t-th step either being masked with a masking ratio $(1-\alpha_t)$ or the same as the original protein sequence x(0).

During the training of the DPLM 210, the discrete probability diffusion is associated with the training of the autoregressive language model and the mask language model, but the learning objective of discrete probability diffusion can be further simplified. In some embodiments, reweighted cross-entropies into which are reparameterized backward transition, from KL divergences between two categoricals, which is expressed as:

$$\mathcal{J}_t = \mathbb{E}_{q(\mathbf{x}^{(0)})} - \mathrm{KL}\left[q(\mathbf{x}^{(t-1)}|\mathbf{x}^{(t)}, \mathbf{x}^{(0)})\|p_\theta(\mathbf{x}^{(t-1)}|\mathbf{x}^{(t)})\right] \tag{6}$$

$$= \mathbb{E}_{q(\mathbf{x}^{(0)})}\left[\lambda^{(t)}\sum_{1\le i\le L}b_i(t)\cdot\log p_\theta(\mathbf{x}_i^{(0)}|\mathbf{x}^{(t)})\right]$$

where $\lambda^{(t)}$ is a weighting coefficient induced from the specific noising schedule. It can be seen from the above formula (6) that the mask language model in the case of $\mathbf{x}^{(t)} \triangleq \bar{\mathbf{x}}_{\mathrm{m}}$ in the above formula (1) and the autoregressive language model in the case of $\mathbf{x}^{(t)} \triangleq \mathbf{x}_{<t}$ and $b_i \triangleq 1$ in the above formula (2) can be considered as special use cases in the generalized form of the discrete diffusion language model. Therefore, according to the learning process of formula (6), the learning process of the mask language model and the autoregressive language model is included, which means that the learned model can have the understanding and prediction capability provided by the mask language model at the same time, and the sequence generation capability provided by the autoregressive language model.

On the basis of the model structure discussed above, the DPLM 210 in embodiments of the present disclosure is also pre-trained on a large-scale real protein sequence set. The real protein sequence set includes an evolutionary level protein sequence 220, i.e., a protein sequence that has been detected in the real world. In the pre-training process, the forward diffusion process starts from the real protein sequence $x^{(0)}$ in the pre-training data set and is gradually noised through $T$ steps until a stationary noise distribution is obtained, that is, all elements in the sequence are represented as [X]. The backward denoising process is started from the stationary noise distribution $x^{(T)}$, and is gradually reduced until a real protein sequence $x^{(0)}$ in the pre-trained data set can be generated.

By pre-training on a large number of real protein sequences, the DPLM 210 can learn a feature representation of the real protein sequence and learn how to generate a protein sequence that conforms to the true protein sequence characteristics. For a given trained model, it can synthesize a new protein sequence through the inverse iterative denoising process of discrete diffusion. Finally, the discrete diffusion may be sampled from the following distribution:

$$p_\theta(\mathbf{x}^{(t-1)}|\mathbf{x}^{(t)}) = \sum_{\hat{\mathbf{x}}_0}q(\mathbf{x}^{(t-1)}|\mathbf{x}^{(t)}, \hat{\mathbf{x}}_0)p_\theta(\hat{\mathbf{x}}_0|\mathbf{x}^{(t)}) \tag{7}$$

In particular, at t-th step, $\hat{\mathbf{x}}_0$ is first generated from $p_\theta(\cdot|\mathbf{x}^{(t)})$, then a less noisy $\mathbf{x}^{(t-1)}$ is obtained $q(\cdot|\mathbf{x}^{(t)}, x^{(0)} = \hat{\mathbf{x}}_0)$ by given $\mathbf{x}^{(t)}$ and $\hat{\mathbf{x}}_0$. This process is repeated from $T$ to 1.

The generative denoising process of the DPLM 210 may be considered an iterative mask prediction process. Specifically, the initial sequence may be a sequence with all noisy states (i.e., all [X]). In each iteration, according to $\log p_\theta$ $(\hat{\mathbf{x}}_0|\mathbf{x}^{(t)})$, one or more elements in the initial sequence are updated based on the prediction $\hat{\mathbf{x}}_0$ of the model, i.e., where one or more elements are updated from the noisy state to indicate a certain amino acid, while the remaining elements remain in the noisy state.

In some embodiments, the DPLM 210 may support the generation of a new protein sequence starting with a random input sequence in the form of protein sequence. An input sequence in the form of protein sequence may be input to the DPLM 210, which generates a first target protein sequence generated by the target model. That is, during an entire backward denoising process, the DPLM 210 may support denoising starting from arbitrary sequence data, and finally generate a protein sequence. Thus, for a protein sequence generation task, the DPLM 210 may be used to generate a target protein sequence based at least on an input sequence in the form of protein sequence. In some embodiments, a random input sequence may be user input or specified by other approaches. All elements in the random input sequence may indicate a noisy state, or some elements therein indicate a noisy state while other elements indicate one or more amino acids.

In some embodiments, the generated protein sequence may be used to predict the corresponding protein structure 230. Specifically, various protein structure prediction methods or tools may be used, including but not limited to an ESMFold tool, to predict the 3D protein structure from the protein sequence.

In supporting the representation learning aspect of the predictive task, the DPLM 210 is able to denoise the input protein sequence at various noise reduction levels, including the original noise-free protein sequence. Thus, the DPLM 210 can be used as a protein sequence representation learner for a large amount of protein sequence data at the same time. Given a target protein sequence, the DPLM 210 may extract a sequence feature representation (also referred to as a sequence embedding representation) of the target protein sequence. The sequence feature representation may be used for various downstream prediction tasks, including sequence level or residue level classification tasks or regression tasks. The extraction of the sequence feature representation may be for the DPLM 210 using a given target protein sequence x as an input for feature extraction, which is represented as:

$$\mathbf{h}(\mathbf{x}) \leftarrow \mathrm{DPLM}_{\theta}(\mathbf{x}, t = 0) \in \mathbb{R}^{L \times d} \qquad (8)$$

where d is the dimension of the sequence feature representation, and $\mathbf{h}(x)$ is the extracted feature representation sequence.

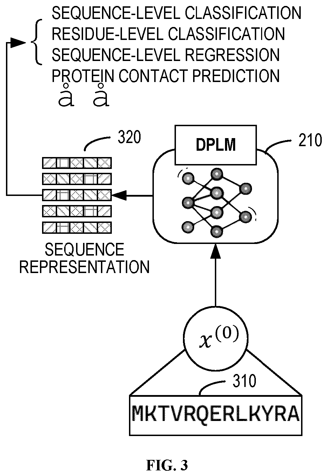

FIG. 3 illustrates an example of extracting a sequence feature representation of a protein sequence with a model according to some embodiments of the present disclosure. For a given target protein sequence 310, it may be considered an initial state in the DPLM 210, i.e., t = 0. Then, the target protein sequence 310 is input to the DPLM 210, and the sequence feature representation 320 corresponding to the target protein sequence 310 is obtained by processing as above formula (8).

In some embodiments, the sequence feature representation corresponding to the extracted target protein sequence 310 may be used to perform a classification task or regression task at a sequence level or a residue level associated with the target protein sequence 310, including but not limited to, a residue classification task, a sequence classification task, a sequence regression task, a contact point prediction task, and the like. Since the DPLM 210 is able to accurately understand and extract the feature representation of the protein sequence, this will significantly improve result accuracy of subsequent downstream prediction tasks.

In some embodiments, the DPLM 210 may also support conditioning generation tasks. In some application scenarios, it may be desirable to generate a protein sequence that satisfies certain conditions. Such conditioning generation tasks include sequence conditioning, cross-modal conditioning, and plug-and-play preference-guided conditioning.

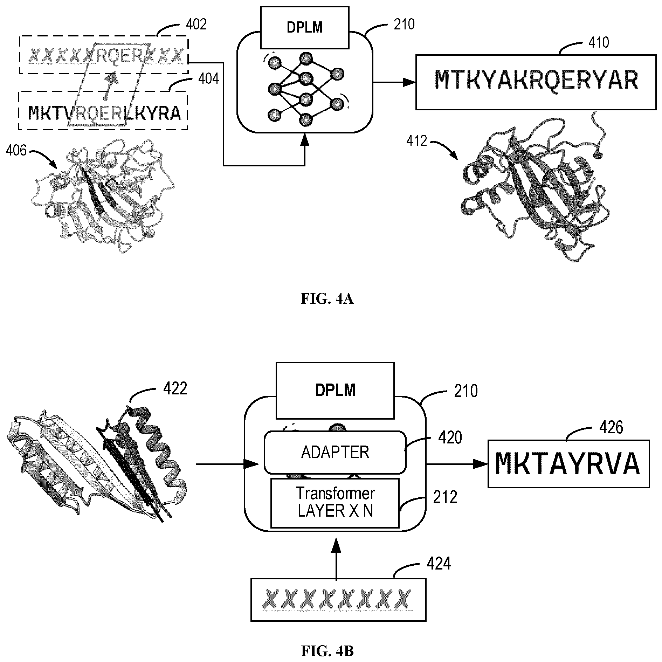

FIG. 4A illustrates an example of conditional generation task conditioned on partial sequences according to some embodiments of the present disclosure. In conditioning generation task with conditioning on partial sequence, the input sequence of the DPLM 210 includes a specified input sequence in the form of protein sequence, elements at at least one position in the specified input sequence indicates at least one specified amino acid. In practical application scenarios, the generation task conditioning on partial sequence is applicable in generating scaffolds for given functional motifs, infilling antibody CDR loops, or imposing expert knowledge a-priori condition. In these application scenarios, the DPLM 210 needs to sample from a conditional distribution $\mathbf{x} \sim p_{\theta}(\mathbf{x}|\bar{\mathbf{x}}) = \prod_{i=1}^{L} b_i \cdot p_{\theta}(x_i|\bar{\mathbf{x}})$ where $\bar{\mathbf{x}}$ represents a

specified input sequence for partial amino acids as. If for individual elements in the sequence $\bar{\mathbf{x}}$, if $b_i = 0$, $\bar{x}_i \in \mathcal{V}$, if $b_i = 1|i \in [1,L]$, $\bar{x}_{i=}$ [X] That is, $b_i \in \{0,1\}$ indicates whether the predicted sequence must preserve the designation of the i-th residue so to output elements $x_i = \bar{x}_i$ in the protein sequence $\mathbf{x}$.

As shown in FIG. 4A, in some application scenarios, for a protein sequence 404 corresponding to a known protein structure 406, it is desirable to obtain a new protein sequence that is the same as amino acid(s) at certain position(s) in the protein sequence 404. In this case, a specified input sequence 402 may be constructed, and elements at corresponding positions indicate amino acids at the same position in the protein sequence 404. The specified input sequence 402 is provided to the DPLM 210 as an input. Through the processing of the DPLM 210, the amino acids at certain positions in a generated target protein sequence 410 correspond to the specified amino acids at the same position in the protein sequence 404. From the target protein sequence 410, a corresponding new protein structure 412 may be generated.

In some embodiments, a cross-modal conditioned generation refers to introducing non-sequence modal protein data (protein data of non-sequential modality) as a constraint in protein sequence generation. The process of generating the protein sequence is constrained by cross-modal constraints $c$, that is $\mathbf{x} \sim p_{\theta}(\mathbf{x}|c)$. For example, in inverse protein folding, it is desirable to generate a protein sequence for a given backbone structure (also referred to as a protein secondary structure). Considering that the DPLM 210 is operating on a sequence that takes amino acids as a token, in this case, additional adapters for the DPLM 210 need to be additionally configured for supporting the cross-modal conditioning generation.

FIG. 4B illustrates an example of a conditioning generation task conditioned on a secondary structure according to some embodiments of the present disclosure. As shown in FIG. 4B, an adapter 420 is introduced into the DPLM 210, which is connected to an existing Transformer layer 212 in the DPLM 210. In this case, the pre-trained DPLM 210 is considered as a modal encoder $\varepsilon(c)$. The adaptor 420 is configured to predict a corresponding protein sequence based on a sequence feature representation extracted by the modal encoder $\varepsilon(c)$ and input non-sequence modal protein data.

In the training process, the pre-trained DPLM 210 may be fixed to keep parameters unchanged. The adapter 420 is then trained based on supervised training. Training data of the supervised training includes non-sequence modal sample protein data and protein sequence(s) corresponding to the sample protein data. In some embodiments, the non-sequence modal protein data includes a protein secondary structure. During training the adapter 420, in order to support the supervised training, a certain number of pairs of sample protein secondary structure and corresponding protein sequence need to be collected, which is expressed as ($\mathbf{x}$, $\mathbf{c}$), where the cross-modal constraint c represents a protein secondary structure, and x represents a corresponding protein sequence.

Since the DPLM 210 has been pre-trained, only a small number of pairs of training samples ($\mathbf{x}$, $\mathbf{c}$) are required in the supervised training process of the adapter 420 to complete the training of the adapter 420. The DPLM 210 with the cross-modal adapter 420 may be represented as a conditional DPLM $p_\theta(\mathbf{x}|\varepsilon(\mathbf{c}))$.

After completion of the training, the input sequence that is input to the DPML with the cross-modal adapter 420 may include a random input sequence in the form of protein sequence or a specified input sequence (i.e., the amino acid of the partial sequence is specified). FIG. 4B illustrates that a random input sequence 424 is input to the DPLM 210. Further, the DPLM 210 will act as a modal encoder to extract the random input sequence or specify a sequence feature representation of the input sequence.

In addition, non-sequence modal input protein data, such as protein secondary structure 422, is input to the adapter 420. The adaptor 420 generates the target protein sequence 426 corresponding to the protein secondary structure 422 based on the non-sequence modal input protein data (i.e., the protein secondary structure 422) and the sequence feature representation extracted by the Transformer layer 212. The target protein sequence 426 may be folded to have the protein secondary structure 422.

It should be understood that, in addition to the protein secondary structure, on the basis of the pre-trained DPLM 210, an adapter adapted to other non-sequence modal protein data may be trained.

In some embodiments, in protein sequence generation, it may also be desirable to support a plug-and-play controllable sequence generation task. In general, it is relatively difficult to directly construct conditioning generation models. Thus, introducing classifier guidance in a continuous diffusion model is a way to implement a conditioning generation model by utilizing the pre-trained classifier to guide the generation process toward desired preference information. However, the prerequisite for continuous classifier guidance is log derivable, i.e., $\nabla_\mathbf{x} \log p_\theta(x)$. This will not hold true for the discrete diffusion process.

In some embodiments of the present disclosure, to support controllable sequence generation in a discrete diffusion model structure, it is proposed to introduce discrete classifier guidance in the pre-trained DPLM 210 to achieve such controllable generation tasks.

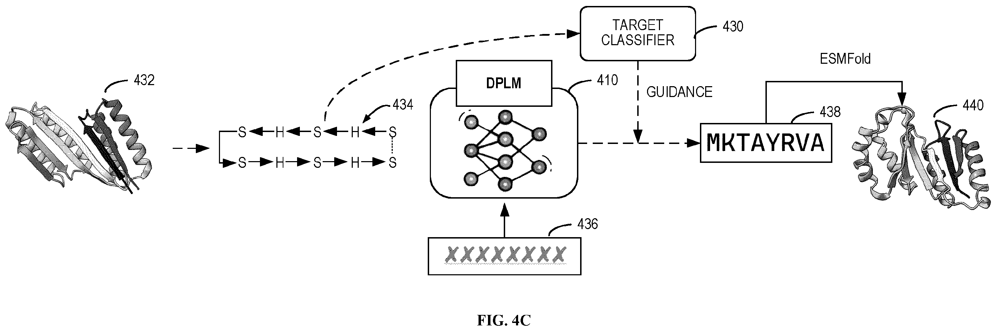

FIG. 4C illustrates an example of a controllable sequence generation task with guidance information according to some embodiments of the present disclosure. The input sequence to be processed by the DPLM 210 may include a random input sequence in the form of protein sequence or a specified input sequence. FIG. 4C illustrates that a random input sequence 436 is input to the DPLM 210. In the protein sequence generation process, the reference property category corresponding to the reference protein data is determined using the trained target classifier 430.

The reference protein data may correspond to protein data under any property. As an example, the reference protein data includes a protein secondary structure. In the example of FIG. 4C, annotation information 434 for a corresponding protein secondary structure may be determined from the reference protein structure 432. In this example, the target classifier 430 is also referred to as a secondary structure classifier that classifies the annotation information 434 of the protein secondary structure to classify it into one of the plurality of protein secondary structure categories.

During the sequence generation of the DPLM 210, the target protein sequence 438 is generated using the DPLM 210 based on an input sequence in the form of protein sequence under the guidance of the reference property category (for example, the protein secondary structure category). The target protein sequence 438 will have a reference property category as the guidance information, although the target protein sequence may be different from the protein sequence corresponding to the protein structure 432.

In addition to the protein secondary structure, other properties of the protein may also be used as guidance information to guide the DPLM 210 to generate a protein sequence with the same property category. In this way, the corresponding protein sequence can be conveniently generated according to the preference. In this solution, different classifiers can be conveniently introduced to classify protein data of the corresponding property.

In a conditioning generation process based on the guidance information, it is desirable to attempt to sampling from a conditional distribution $q(\mathbf{x}^{(t-1)}|\mathbf{x}^{(t)},\mathbf{y}) \propto q(\mathbf{x}^{(t-1)}|\mathbf{x}^{(t)})q(\mathbf{y}|\mathbf{x}^{(t-1)})$, where $\mathbf{y}$ represents guidance information as a condition (also referred to as preference information), such conditional distribution approximates $p_\theta(\mathbf{x}^{(t-1)}|\mathbf{x}^{(t)})p_\phi(\mathbf{y}|\mathbf{x}^{(t-1)})$, where $p_\phi(\mathbf{y}|\mathbf{x}^{(t-1)})$ is a discriminative guidance model (for example, a classifier or regressor for guidance information input by a user). However, $p_\phi(\mathbf{y}|\mathbf{x}^{(t-1)})$ cannot be factorized over all positions, prohibiting evaluation of all possible values of $\mathbf{x}^{(t-1)}$. To this end, in embodiments of the present disclosure, the sequence data $\mathbf{x}$ associated with the discrete protein sequence is first considered to be a continuous random variable in the DPLM 210, and the first-order Taylor expansion is performed on $\mathbf{x}^{(t)}$ to obtain:

$$\log q(\mathbf{y}|\mathbf{x}^{(t-1)}) \tag{9}$$

$$\approx \log q(\mathbf{y}|\mathbf{x}^{(t)}) + \langle \nabla_{\mathbf{x}} \log q(\mathbf{y}|\mathbf{x}^{(t)}), \mathbf{x}^{(t-1)} - \mathbf{x}^{(t)} \rangle$$

$$\approx \sum_{1 \le i \le L} \langle \nabla_{\mathbf{x}_i} \log q(\mathbf{y}|\mathbf{x}^{(t)}), \mathbf{x}_i^{(t-1)} \rangle + C(\mathbf{x}^{(t)}),$$

where $C(\mathbf{x}^{(t)})$ is a constant that is not dependent on $\mathbf{x}^{(t-1)}$. $p_\phi(\mathbf{y}|\mathbf{x}^{(t)})$ is used to estimate $q(\mathbf{y}|\mathbf{x}^{(t)})$ and plug it into the above formula (8). This may be sampled from the conditional distribution instead of sampling at each step t, which may be represented as follows:

$$\mathbf{x}^{(t-1)} \sim p_\theta(\mathbf{x}^{(t-1)}|\mathbf{x}^{(t)}) p_\phi(\mathbf{y}|\mathbf{x}^{(t-1)})^\eta \tag{10}$$

$$\propto p_\theta(\mathbf{x}^{(t-1)}|\mathbf{x}^{(t)}) e^{\left( \eta \cdot \sum_i \langle \nabla_{\mathbf{x}_i} \log p_\phi(\mathbf{y}|\mathbf{x}^{(t)}), \mathbf{x}_{(i)}^{t-1} \rangle \right)}$$

where $\eta$ is a tunable parameter for controlling the controlling degree of the guidance information on the sequence generation.

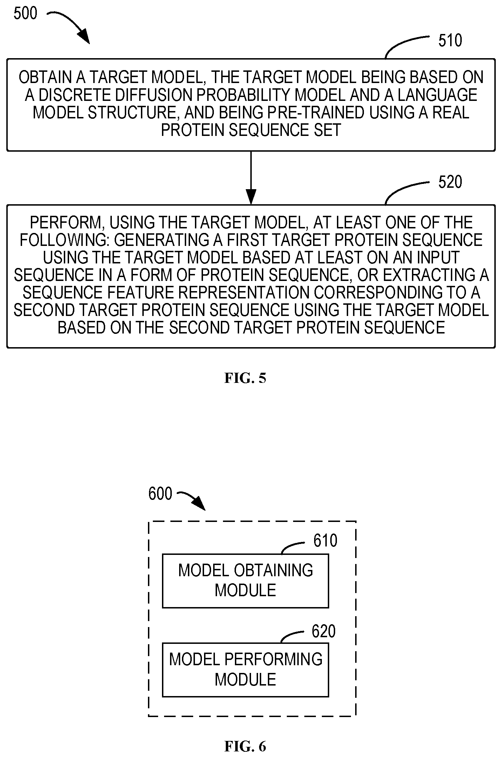

FIG. 5 shows a flowchart of a processing process for protein related information according to some embodiments of the present disclosure . For ease of discussion, the process 500 will be described with reference to the environment 100 of FIG. 1. Process 500 may be implemented at the protein-related processing system 110. As mentioned above, the protein-related processing system 110 may be a server device or a terminal device, which is not limited in the scope of the embodiments of the present disclosure.

At block 510, the processing system 110 obtains a target model that is based on a discrete diffusion probability model and a language model structure and is pre-trained with a real protein sequence set.

At block 520, the processing system 110 utilizes the target model to perform at least one of the following: generating a first target protein sequence using the target model based at least on an input sequence in a form of protein sequence, or extracting a sequence feature representation corresponding to a second target protein sequence using the target model based on the second target protein sequence.

In some embodiments, the input sequence includes a random input sequence in the form of protein sequence. Generating the first target protein sequence using the target model based at least on the input sequence in the form of protein sequence includes: providing the random input sequence to the target model to generate a first target protein sequence generated by the target model.

In some embodiments, the input sequence includes a specified input sequence in the form of protein sequence, and an element at at least one position in the specified input sequence indicates at least one specified amino acid. Generating the first target protein sequence using the target model based at least on the input sequence in the form of protein sequence includes: providing the specified input sequence to the target model to generate a first target protein sequence output by the target model, an element at at least one position in the first target protein sequence indicating the at least one specified amino acid.

In some embodiments, the input sequence includes a random input sequence or a specified input sequence in the form of protein sequence. Generating the first target protein sequence using the target model based at least on the input sequence in the form of protein sequence includes: determining a reference property category corresponding to the reference protein data using a trained target classifier; and based on the input sequence in the form of protein sequence, generating a first target protein sequence using the target model under a guidance of the reference property category, the first target protein sequence having the reference property category.

In some embodiments, the reference protein data includes a protein secondary structure, and the reference property category includes one of a plurality of protein secondary structure categories.

In some embodiments, the process 500 further includes: in response to that the pre-trained target model is fixed, training an adapter based on supervised training, the adapter being configured to predict a corresponding protein sequence based on a sequence feature representation and non-sequence modal protein data, and training data of the supervised training including non-sequence modal sample protein data and a protein sequence corresponding to the sample protein data.

In some embodiments, the input sequence includes a random input sequence in the form of protein sequence or a specified input sequence. Generating the first target protein sequence using the target model based at least on the input sequence in the form of protein sequence includes: providing the random input sequence or the specified input sequence to the target model to extract a sequence feature representation of the random input sequence or the specified input sequence; and generating, using the adapter, a first target protein sequence corresponding to the input protein data based on non-sequence modal input protein data and the sequence feature representation.

In some embodiments, the non-sequence modal protein data includes a protein secondary structure.

In some embodiments, the process 500 further includes based on a sequence feature representation corresponding to the second target protein sequence, performing at least one of the following tasks: a residue classification task for the second target protein sequence, a sequence classification task for the second target protein sequence, a sequence regression task for the second target protein sequence, and a contact point prediction task for the second target protein sequence.

Embodiments of the present disclosure also provide a corresponding apparatus for implementing the above method or process. FIG. 6 shows a block diagram of an apparatus 600 for document query according to some embodiments of the present disclosure. The apparatus 600 may be implemented or included in a protein-related processing system 110. The various modules/components in the apparatus 600 may be implemented by hardware, software, firmware, or any combination thereof.

As shown in FIG. 6, the apparatus 600 includes a model obtaining module 610 configured to obtain a target model which is based on a discrete diffusion probability model and a language model structure and is pre-trained by using a real protein sequence set. The apparatus 600 further includes a model performing module 620 configured to perform, by using the target model, at least one of the followings: generating a first target protein sequence using the target model based at least on an input sequence in a form of protein sequence, or extracting a sequence feature representation corresponding to a second target protein sequence using the target model based on the second target protein sequence.

In some embodiments, the input sequence includes a random input sequence in the form of protein sequence. Generating the first target protein sequence using the target model based at least on the input sequence in the form of protein sequence includes: providing the random input sequence to the target model to generate a first target protein sequence generated by the target model.

In some embodiments, the input sequence includes a specified input sequence in the form of protein sequence, and an element at at least one position in the specified input sequence indicates at least one specified amino acid. The model performing module 620 is configured to provide the specified input sequence to the target model to generate a first target protein sequence output by the target model, an element at at least one position in the first target protein sequence indicating the at least one specified amino acid.

In some embodiments, the input sequence includes a random input sequence or a specified input sequence in a form of a protein sequence. The model performing module 620 is configured to: determine a reference property category corresponding to the reference protein data using a trained target classifier; and based on the input sequence in the form of protein sequence, generate a first target protein sequence using the target model under a guidance of the reference property category, the first target protein sequence having the reference property category.

In some embodiments, the reference protein data includes a protein secondary structure, and the reference property category includes one of a plurality of protein secondary structure categories.

In some embodiments, the apparatus 600 further includes an adapter training module configured to: in response to that the pre-trained target model is fixed, train an adapter based on supervised training, the adapter being configured to predict a corresponding protein sequence based on a sequence feature representation and a non-sequence modal protein data, and training data of the supervised training including non-sequence modal sample protein data and a protein sequence corresponding to sample protein data.

In some embodiments, the input sequence includes a random input sequence in a form of protein sequence or a specified input sequence. The model performing module 620 is configured to: provide the random input sequence or the specified input sequence to the target model to extract a sequence feature representation of the random input sequence or the specified input sequence; and generate, using the adapter, a first target protein sequence corresponding to the input protein data based on non-sequence modal input protein data and the sequence feature representation .

In some embodiments, the non-sequence modal protein data includes a protein secondary structure.

In some embodiments, the apparatus 600 further includes a downstream task performing module configured to based on a sequence feature representation corresponding to the second target protein sequence, performing at least one of the following tasks: a residue classification task for the second target protein sequence, a sequence classification task for the second target protein sequence, a sequence regression task for the second target protein sequence, and a contact point prediction task for the second target protein sequence.

The units and/or modules included in the apparatus 600 may be implemented in various manners, including software, hardware, firmware, or any combination thereof. In some embodiments, one or more units and/or modules may be implemented using software and/or firmware, such as machine-executable instructions stored on a storage medium. In addition to or as an alternative to machine-executable instructions, some or all of the units and/or modules in the apparatus 600 may be implemented, at least in part, by one or more hardware logic components. By way of example and not limitation, example types of hardware logic components that may be used including field programmable gate arrays (FPGAs), application specific integrated circuits (ASICs), application specific standards (ASSPs), system-on-a-chip (SOCs), complex programmable logic devices (CPLDs), and the like.

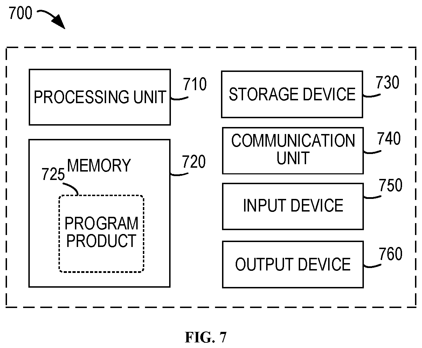

FIG. 7 illustrates a block diagram of an electronic device 700 in which one or more embodiments of the present disclosure may be implemented. It should be understood that the electronic device 700 illustrated in FIG. 7 is merely example and should not constitute any limitation on the functionality and scope of the embodiments described herein. The electronic device 700 shown in FIG. 7 may be configured to implement the protein-related processing system 110 of FIG. 1 or the device 600 of FIG. 6.

As shown in FIG. 7, the electronic device 700 is in the form of a general-purpose electronic device. Components of the electronic device 700 may include, but are not limited to, one or more processors or processing units 710, a memory 720, a storage device 730, one or more communication units 740, one or more input devices 750, and one or more output devices 760. The processing unit 710 may be an actual or virtual processor and capable of performing various processes according to programs stored in the memory 720. In multiprocessor systems, multiple processing units execute computer-executable instructions in parallel to improve parallel processing capabilities of electronic device 700.

Electronic device 700 typically includes a plurality of computer storage media. Such media may be any available media accessible to the electronic device 700, including, but not limited to, volatile and non-volatile media, removable and non-removable media. The memory 720 may be volatile memory (e.g., registers, caches, random access memory (RAM)), non-volatile memory (e.g., read-only memory (ROM), electrically erasable programmable read-only memory (EEPROM), flash memory), or some combination thereof. Storage device 730 may be a removable or non-removable medium and may include a machine-readable medium, such as a flash drive, magnetic disk, or any other medium, which may be capable of storing information and/or data and may be accessed within electronic device 700.

The electronic device 700 may further include additional removable / non-removable, volatile / non-volatile storage media. Although not shown in FIG. 7, a disk drive for reading or writing from a removable, nonvolatile magnetic disk (e.g., a "floppy disk") and an optical disk drive for reading or writing from a removable, nonvolatile optical disk may be provided. In these cases, each drive may be connected to a bus (not shown) by one or more data media interfaces. The memory 720 may include a computer program product 725 having one or more program modules configured to perform various methods or actions of various embodiments of the present disclosure.

The communication unit 740 is configured to communicate with another electronic device through a communication medium. Additionally, the functionality of components of the electronic device 700 may be implemented in a single computing cluster or multiple computing machines capable of communicating over a communication connection. Thus, the electronic device 700 may operate in a networked environment using logical connections with one or more other servers, network personal computers (PCs), or another network node.

The input device 750 may be one or more input devices, such as a mouse, a keyboard, a trackball, or the like. The output device 760 may be one or more output devices, such as a display, a speaker, a printer, or the like. The electronic device 700 may also communicate with one or more external devices (not shown) through the communication unit 740 as needed, external devices such as storage devices, display devices, etc. , communicate with one or more devices that enable a user to interact with the electronic device 700, or communicate with any device (e.g., a network card, a modem, etc. ) that enables the electronic device 700 to communicate with one or more other electronic devices. Such communication may be performed via an input / output (I / O) interface (not shown).

According to example implementations of the present disclosure, there is provided a computer-readable storage medium having computer-executable instructions stored thereon, wherein the computer-executable instructions are executed by a processor to implement the method described above. According to example implementations of the present disclosure, a computer program product is further provided, the computer program product being tangibly stored on a non-transitory computer-readable medium and including computer-executable instructions, the computer-executable instructions being executed by a processor to implement the method described above.

Aspects of the present disclosure are described herein with reference to flowcharts and / or block diagrams of methods, apparatuses, devices, and computer program products implemented in accordance with the present disclosure. It should be understood that each block of the flowchart and / or block diagram, and combinations of blocks in the flowcharts and / or block diagrams, may be implemented by computer readable program instructions.

These computer-readable program instructions may be provided to a processing unit of a general purpose computer, special purpose computer, or other programmable data processing apparatus to produce a machine, such that the instructions, when executed by a processing unit of a computer or other programmable data processing apparatus, produce means to implement the functions / acts specified in the flowchart and / or block diagram. These computer-readable program instructions may also be stored in a computer-readable storage medium that cause the computer, programmable data processing apparatus, and / or other devices to function in a particular manner, such that the computer-readable medium storing instructions includes an article of manufacture including instructions to implement aspects of the functions / acts specified in the flowchart and / or block diagram (s).

The computer-readable program instructions may be loaded onto a computer, other programmable data processing apparatus, or other apparatus, such that a series of operational steps are performed on a computer, other programmable data processing apparatus, or other apparatus to produce a computer-implemented process such that the instructions executed on a computer, other programmable data processing apparatus, or other apparatus implement the functions / acts specified in the flowchart and / or block diagram block or blocks.

The flowchart and block diagrams in the figures show architecture, functionality, and operation of possible implementations of systems, methods, and computer program products according to various implementations of the present disclosure. In this regard, each block in the flowchart or block diagram may represent a module, program segment, or portion of an instruction that includes one or more executable instructions for implementing the specified logical function. In some alternative implementations, the functions noted in the blocks may also occur in a different order than noted in the figures. For example, two consecutive blocks may actually be performed substantially in parallel, which may sometimes be performed in the reverse order, depending on the functionality involved. It is also noted that each block in the block diagrams and / or flowchart, as well as combinations of blocks in the block diagrams and / or flowchart, may be implemented with a dedicated hardware-based system that performs the specified functions or actions, or may be implemented in a combination of dedicated hardware and computer instructions.

Various implementations of the present disclosure have been described above, which are exemplary, not exhaustive, and are not limited to the implementations disclosed. Many modifications and variations will be apparent to those of ordinary skill in the art without departing from the scope and spirit of the various implementations illustrated. The selection of the terms used herein is intended to best explain the principles of the implementations, practical applications, or improvements to techniques in the marketplace, or to enable others of ordinary skill in the art to understand the various implementations disclosed herein.

**Claims**

1. A method for information processing, comprising:

   obtaining a target model which is based on a discrete diffusion probability model and a language model structure and is pre-trained using a real protein sequence set; and
   performing, using the target model, at least one of the following:

   generating a first target protein sequence using the target model based at least on an input sequence in a form of protein sequence, or
   extracting a sequence feature representation corresponding to a second target protein sequence using the target model based on the second target protein sequence.

2. The method of claim 1, wherein the input sequence comprises a random input sequence in a form of protein sequence, and wherein generating the first target protein sequence using the target model based at least on the input sequence in the form of protein sequence comprises:
   providing the random input sequence to the target model to generate a first target protein sequence generated by the target model.

3. The method of claim 1, wherein the input sequence comprises a specified input sequence in a form of protein sequence, an element at at least one position in the specified input sequence indicates at least one specified amino acid, and wherein generating the first target protein sequence using the target model based at least on the input sequence in the form of protein sequence comprises:
   providing the specified input sequence to the target model to generate a first target protein sequence output by the target model, an element at at least one position in the first target protein sequence indicating the at least one specified amino acid.

4. The method of claim 1, wherein the input sequence comprises a random input sequence or a specified input sequence in a form of protein sequence, and wherein generating the first target protein sequence using the target model based at least on the input sequence in the form of protein sequence comprises:

   determining a reference property category corresponding to reference protein data using a trained target classifier; and
   based on the input sequence in the form of protein sequence, generating a first target protein sequence using the target model under a guidance of the reference property category, the first target protein sequence having the reference property category.

5. The method of claim 4, wherein the reference protein data comprises a protein secondary structure and the reference property category comprises one of a plurality of protein secondary structure categories.

6. The method of claim 1, further comprising:
in response to that the pre-trained target model is fixed, training an adapter based on supervised training, the adapter being configured to predict a corresponding protein sequence based on a sequence feature representation and a non-sequence modal protein data, and training data of the supervised training comprising non-sequence modal sample protein data and a protein sequence corresponding to sample protein data.

7. The method of claim 6, wherein the input sequence comprises a random input sequence or a specified input sequence in a form of protein sequence, and wherein generating the first target protein sequence using the target model based at least on the input sequence in the form of protein sequence comprises:

   providing the random input sequence or the specified input sequence to the target model to extract a sequence feature representation of the random input sequence or the specified input sequence; and
   generating, using the adapter, a first target protein sequence corresponding to the input protein data based on non-sequence modal input protein data and the sequence feature representation.

8. The method of claim 6 or 7, wherein the non-sequence modal protein data comprises a protein secondary structure.

9. The method of any of claims 1-8, further comprising:
based on a sequence feature representation corresponding to the second target protein sequence, performing at least one of the following tasks:

   a residue classification task for the second target protein sequence,
   a sequence classification task for the second target protein sequence,
   a sequence regression task for the second target protein sequence, or
   a contact point prediction task for the second target protein sequence.

10. An apparatus for processing information, comprising:

   an obtaining module configured to obtain a target model which based on a discrete diffusion probability model and a language model structure is being pre-trained using a real protein sequence set; and
   a model performing module configured to perform, by using the target model, at least one of the following:

      generating a first target protein sequence using the target model based at least on an input sequence in a form of protein sequence, or
      extracting a sequence feature representation corresponding to the second target protein sequence using the target model based on the second target protein sequence.

11. An electronic device, comprising:

   at least one processing unit; and
   at least one memory coupled to the at least one processing unit and storing instructions for execution by the at least one processing unit, the instructions, when executed by the at least one processing unit, causing the electronic device to perform the method of any of claims 1-9.

12. A computer-readable storage medium having a computer program stored thereon executable by a processor to implement the method of any of claims 1-9.

13. A computer program product tangibly stored in a computer storage medium and comprising computer-executable instructions that, when executed by a device, cause the device to perform the method of any of claims 1-9.

100

110

102

112

INPUT

PROTEIN
RELATED
PROCESSING
SYSTEM

PROTEIN
SEQUENCE

114

SEQUENCE
REPRESENTATION

120

TARGET
MODEL

**FIG. 1**

FIG. 2

SEQUENCE-LEVEL CLASSIFICATION
RESIDUE-LEVEL CLASSIFICATION
SEQUENCE-LEVEL REGRESSION
PROTEIN CONTACT PREDICTION

320

**DPLM** 210

SEQUENCE
REPRESENTATION

$x^{(0)}$

310

MKTVRQERLKYRA

**FIG. 3**

**FIG. 4A**

**FIG. 4B**

FIG. 4C

500 ⌐

510

OBTAIN A TARGET MODEL, THE TARGET MODEL BEING BASED ON A DISCRETE DIFFUSION PROBABILITY MODEL AND A LANGUAGE MODEL STRUCTURE, AND BEING PRE-TRAINED USING A REAL PROTEIN SEQUENCE SET

520

PERFORM, USING THE TARGET MODEL, AT LEAST ONE OF THE FOLLOWING: GENERATING A FIRST TARGET PROTEIN SEQUENCE USING THE TARGET MODEL BASED AT LEAST ON AN INPUT SEQUENCE IN A FORM OF PROTEIN SEQUENCE, OR EXTRACTING A SEQUENCE FEATURE REPRESENTATION CORRESPONDING TO A SECOND TARGET PROTEIN SEQUENCE USING THE TARGET MODEL BASED ON THE SECOND TARGET PROTEIN SEQUENCE

**FIG. 5**

600

610

MODEL OBTAINING MODULE

620

MODEL PERFORMING MODULE

**FIG. 6**

700

PROCESSING UNIT — 710

STORAGE DEVICE — 730

725 MEMORY — 720

PROGRAM PRODUCT

COMMUNICATION UNIT — 740

INPUT DEVICE — 750

OUTPUT DEVICE — 760

**FIG. 7**

## INTERNATIONAL SEARCH REPORT

| International application No. |
| --- |
| **PCT/CN2024/077839** |

**A.      CLASSIFICATION OF SUBJECT MATTER**

G16B 30/00(2019.01)i;  G16B5/20(2019.01)i;  G16B40/00(2019.01)i;  G06N3/08(2023.01)i

According to International Patent Classification (IPC) or to both national classification and IPC

**B.      FIELDS SEARCHED**

Minimum documentation searched (classification system followed by classification symbols)

IPC:G16B30/-; G16B5/-; G16B40/-; G06N3/-

Documentation searched other than minimum documentation to the extent that such documents are included in the fields searched

Electronic data base consulted during the international search (name of data base and, where practicable, search terms used)

CNABS, CNTXT, ENTXTC, ENTXT, CNKI, 万方, WANFANG, web of science, IEEE, 百度学术, BAIDU SCHOLAR, 必应检索, BING: 蛋白质, 序列, 生成, 扩散概率, 语言模型, 训练, 类别, 引导, 参考, 属性, protein, sequence, generate, DDPM, language model, transformer, train, guide, reference, attribute

**C.      DOCUMENTS CONSIDERED TO BE RELEVANT**

| Category* | Citation of document, with indication, where appropriate, of the relevant passages | Relevant to claim No. |
| --- | --- | --- |
| Y | CN 116994642 A (SHANGHAI JIAO TONG UNIVERSITY) 03 November 2023 (2023-11-03)<br>      description, paragraphs 37-98 | 1-3, 6-13 |
| Y | CN 116978450 A (TENCENT TECHNOLOGY (SHENZHEN) CO., LTD.) 31 October 2023 (2023-10-31)<br>      description, paragraphs 26, 143, 185, and 244 | 1-3, 6-13 |
| A | CN 116580764 A (HANGZHOU CARBONSILICON AI TECHNOLOGY DEVELOPMENT CO., LTD. et al.) 11 August 2023 (2023-08-11)<br>      entire document | 1-13 |
| A | US 2023377690 A1 (ANAND, Namrata) 23 November 2023 (2023-11-23)<br>      entire document | 1-13 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **11 October 2024** | **12 October 2024** |

| Name and mailing address of the ISA/CN | Authorized officer |
| --- | --- |
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/077839**

| Patent document cited in search report | | | Publication date (day/month/year) | Patent family member(s) | Publication date (day/month/year) |
|---|---|---|---|---|---|
| CN | 116994642 | A | 03 November 2023 | None | |
| CN | 116978450 | A | 31 October 2023 | None | |
| CN | 116580764 | A | 11 August 2023 | None | |
| US | 2023377690 | A1 | 23 November 2023 | None | |

Form PCT/ISA/210 (patent family annex) (July 2022)